# EUROPEAN PATENT SPECIFICATION

(11) **EP 2 365 024 B1**
(45) Date of publication and mention of the grant of the patent: **09.07.2014**
(21) Application number: 09830464.5
(22) Date of filing: 04.12.2009
(51) Int. Cl.: C08J 3/12, A61K 8/87, A61Q 13/00, B01J 2/00, B01J 2/04, B01J 20/26, C08G 18/10, C08G 18/48, C09K 3/00, A61L 9/01

(54) **POLYURETHANE PARTICLES AND METHOD FOR PRODUCING POLYURETHANE PARTICLE CLUSTER**
POLYURETHANPARTIKEL UND VERFAHREN ZUR HERSTELLUNG VON POLYURETHANPARTIKELHAUFEN
PARTICULES DE POLYURÉTHANE ET PROCÉDÉ DE FABRICATION D'AGRÉGATS DE PARTICULES DE POLYURÉTHANE

(30) Priority: 05.12.2008 JP 2008310341
(43) Date of publication of application: 14.09.2011
(73) Proprietor: Konishi Co., Ltd., Chuo-ku Osaka-shi Osaka 541-0045 (JP)
(72) Inventor: ORIGUCHI Toshiki, Osaka-shi Osaka 538-0053 (JP); NAKAYAMA Yoshitaka, Osaka-shi Osaka 538-0053 (JP)
(74) Representative: HOFFMANN EITLE
(86) International application number: PCT/JP2009/070378
(87) International publication number: WO 2010/064698

(56) References cited:
- EP-A1- 1 422 259
- EP-A2- 0 616 019
- JP-A- 5 209 017
- JP-A- 6 041 419
- JP-A- 6 041 419
- JP-A- 10 067 958
- JP-A- 10 067 958
- JP-A- 2004 176 066
- JP-A- 2006 008 757
- JP-A- 2006 321 830
- JP-A- 2006 321 830
- JP-A- 2009 029 867

## Description

### Technical Field

The present invention relates to a polyurethane particle and a method for producing polyurethane particles. Especially, the invention relates to the polyurethane particle to superiorly absorb fats and oils such as sebums or oleic acids, and the method for producing them.

### Background Art

In recent years, it is proposed an acrylic polymer particle easy to absorb fats and oils such as sebums or oleic acids. For example, the patent reference 1 discloses a swelling-type oil absorbent polymer particle consisting of a cross-linked acrylic polymer particle which is obtained by polymerizing an alkyl acrylate or alkyl methacrylate which has a liner or branched alkyl group having 18 to 24 carbon atoms, a vinyl monomer which is able to co-polymerize with the alkyl acrylate or alkyl methacrylate, and a cross-linkable monomer.

Patent reference 1: JP2006-8757 (the claims are referred.)

### Summary of Invention

### Technical Problem

The inventors have tried to obtain an oil absorbent particle with using a polyurethane but not acrylic polymer. The inventors have discovered that the polyurethane obtained by selecting a poly(tetramethylene ether)glycol as polyols superiorly absorbs fats and oils such as sebum or oleic acid. Concretely, the inventors have discovered the oil absorbent particle consisting of the polyurethane obtained with the following method. That is, it is obtained an isocyanate-terminated urethane prepolymer by reacting a poly(tetramethylene ether)glycol as polyols and polyisocyanates such as an isophorone diisocyanate. It is obtained an oil-in-water emulsion by dispersing the isocyanate-terminated urethane prepolymer in a water.

A trifunctional or more functional amines is added in the oil-in-water emulsion, and react with the isocyanate-terminated urethane prepolymer in droplets to three-dimensionally polymerize and convert droplets to polyurethane spheres. After that, removing the water of the emulsion with drying, polyurethane particles consisting of the polyurethane spheres are obtained.

However, removing the water of the emulsion with drying, the polyurethane spheres may be easy to cohere in the case the polyurethane sphere is soft or the surface of it is sticky. Furthermore, keeping the obtained polyurethane particles for a long time, the particles may be easy to cohere. The polyurethane particles for oil absorption are generally used as an aggregate of the polyurethane primary particles. Therefore, the polyurethane particles cohering, it is difficult to treat and an oil absorbability lowers because a total surface area of the polyurethane particles is small. Preventing to cohere, the polyurethane particles must be set in strict conditions under producing, treating, keeping and transporting them.

It is therefore the object of the invention to provide polyurethane particles which are difficult to cohere though they are not set in strict conditions under producing, treating, keeping and transporting them. Furthermore, it is the object of the invention to provide methods for producing such polyurethane particles,

### Solution to Problem

The inventors have discovered to solve the problem by making an emulsion in which polyurethane spheres disperses, dispersing hydrophilic fine silica powders in the emulsion and spray-drying the emulsion to remove the water in it. The invention is based on the discovery. The invention is relates to the method for producing polyurethane particles comprising: preparing the hydrophilic fine silica powders, preparing an isocyanate-terminated urethane prepolymer obtained by reacting polyisocyanates and polyols including a poly(tetramethylene ether) glycol, preparing the polyurethane spheres obtained by three-dimensionally polymerizing the isocyanate-terminated urethane prepolymer with trifunctional or more functional amines, preparing a mixed aqueous dispersion by dispersing the hydrophilic fine silica powders and the polyurethane spheres in a water, and spraying the mixed aqueous dispersion in a high temperature atmosphere. Then, the polyurethane particles are obtained by vaporizing the water in the mixed aqueous dispersion, converting the polyurethane spheres to bodies of the polyurethane particles, and covering each surface of the bodies of the polyurethane particles with the hydrophilic fine silica powders. Furthermore, the invention relates to the polyurethane particle. Furthermore, the invention relates to an oil absorbent agent consisting of the polyurethane particles, a perfume comprising the polyurethane particles which hold a perfumed oil therein and chemicals comprising the polyurethane particles which hold therein an oil-based chemical component selected from the group consisting of an oil-based agricultural chemical component, an oil-based attractant component and an oil-based repellent component.

The hydrophilic fine silica powder consists of mainly SiO₂, and has not substantially hydrophobic groups on the surface of it. The hydrophilic fine silica powders are well known, and sold by Nippon Aerosil Co. as AEROSIL^{®} 200, 200V, 200CF, 200FAD, 300, 300CF, 380, 50, 90G, 130, 0X50, MOX80, MOX170, COK84 and etc.. Such hydrophilic fine silica powders are used in the invention. A particle size of the hydrophilic fine silica powder is finer than the particle size of the obtained polyurethane particle to cover the body of the polyurethane particle with the hydrophilic fine silica powders. Concretely, an average particle size of the obtained polyurethane particles is 1-500µ m, and an average particle size of the hydrophilic fine silica powders is 5-40 nm. Therefore, it is preferable that the particle size of the polyurethane particle is 30 or more times larger than the particle size of the hydrophilic fine silica powder. The average particle size of the hydrophilic fine silica powders is decided in nominal value.

It is used as the polyisocyanates for producing a polyurethane to be well known compounds which have two or more isocyanate groups in a molecule. It is preferably used alicyclic polyisocyanates or aliphatic polyisocyanates. Concretly, it is used an isophorone diisocyanate, a 1,6-hexamethylene diisocyanate and a hydrated MDI.

It is used as the polyols for producing a polyurethane to be a poly(tetramethylene ether)glycol. The other polyols may be mixed with the poly(tetramethylene ether)glycol. It may be used as the other polyols to be well known polyols for producing a polyurethane. A number average molecular weight of poly(tetramethylene ether)glycol is not limited, but preferably 650-3000.

Trifunctional or more functional amines for producing a polyurethane are used to three-dimensionally polymerize a urethane prepolymer obtained by reacting the polyols and the polyisocyanates. It is used a diethylenetriamine, triethylenetetramine, tetraethylenepent amine and 3,3'-diaminodipropylamine as the trifunctional or more functional amines.

The polyurethane particle of the invention is obtained by the method described in the above paragraph 0007. The method is the following, describing concretely in the order of processes.

The processes comprise: a process to obtain an isocyanate-terminated urethane prepolymer by reacting polyisocyanates and polyols including a poly(tetramethylene ether)glycol, a process to obtain an oil-in-water emulsion by adding and mixing the isocyanate-terminated urethane prepolymer to an aqueous solution in which a dispersant dissolves, a process to obtain polyurethane spheres in the oil-in-water emulsion by that the isocyanate-terminated urethane prepolymer in oil droplets is three-dimensionally polymerized by adding the trifunctional or more functional amines in the oil-in-water emulsion, a process to obtain an aqueous polyurethane dispersion in which the polyurethane spheres disperse by removing the dispersant from the oil-in-water emulsion, a process to obtain a mixed aqueous dispersion by mixing the aqueous polyurethane dispersion with an aqueous silica dispersion in which hydrophilic fine silica powders disperse in a water, and a process to spray-dry the mixed aqueous dispersion in a high temperature atmosphere with a spray-dryer. Thereby, the water is vaporized in the mixed aqueous dispersion, the polyurethane spheres are converted to bodies of the polyurethane particles, and each surface of the bodies of the polyurethane particles is covered with the hydrophilic fine silica powders.

The processes are more described as the followings. The isocyanate-terminated urethane prepolymer is obtained by reacting conventionally the polyisocyanates and the polyols including a poly(tetramethylene ether)glycol. Then, an amount by mole of NCO group of the polyisocyanates exceeds an amount by mole of OH group of the polyols to terminate at the NCO group in the urethane prepolymer. The amount by mole of NCO group is conventionally 1.5-3.0 times to the amount by mole of OH group. Generally, the polyisocyanates and polyols are dissolved in an organic solvent such as ethyl acetate or methyl ethyl ketone, and react with a tin catalyst such as dibutyl tin dilaurate or an amine catalyst such as 1,8-diazabicyclo[5.4.0]undec-7-ene.

Conventionally, it is used only the poly(tetramethylene ether) glycol as the polyols, but may be mixed with the other polyols. It may be used a polyalcohol, polyetherpolyol, polyesterpolyol, polycarbonatepolyol, polyolefinpolyol,poiyacrylicpolyol or castor oil as the other polyols. Mixing with the other polyols, it is preferable that the poly(tetramethylene ether)glycol occupies 50 or more mass% in the polyols. Less than 50 mass%, the polyurethane particle is insufficient to absorb oils because the poly(tetramethylene ether)glycol is a component to absorb oils. Using only the poly(tetramethylene ether)glycol, the body of the polyurethane particle tends to be soft. Mixing with the polycarbonatepolyol as the other polyols, the body of the polyurethane particle tends to be gradually hard with increase of the content.

The content of the isocyanate group (NCO group) of the isocyanate-terminated urethane prepolymer is 2.0-10 mass%, and preferably 2.5-3.5 mass%. More than 10 mass% in the content, the polyurethane particle is insufficient to absorb oils because a content of urethane linkage is much. Less than 2.0 mass% in the content, it may be difficult to treat the isocyanate-terminated urethane prepolymer because it becomes high molecular weight and high viscosity.

The obtained isocyanate-terminated urethane prepolymer is added and mixed to an aqueous solution in which a dispersant is dissolved to obtain an oil-in-water emulsion. Concretely, the oil-in-water emulsion is obtained by the following method.

It is prepared the aqueous solution in which the dispersant is dissolved. The dispersant is to emulsify the isocyanate-terminated urethane prepolymer in a water. For example, as the dispersant, it is used general surfactants such as nonionic surfactant or usaul polymeric dispersants such as polyvinyl alcohol. The dispersant is dissolved in the water to obtain the aqueous solution. Dissolving the dispersant, it is preferable to heat the water so as to dissolve easily. After the dispersant is entirely dissolved in the water, it is preferable to cool the aqueous solution at room temperature. A temperature of the heated water is decided at a solubility of the dispersant. For example, using the polyvinyl alcohol having a saponification degree of about 86.5-89.0 mol%, the temperature of the heated water is about 90°C . A concentration of the dispersant is decided so as to emulsify the isocyanate-terminated urethane prepolymer in the water. It is conventionally about 3-20 mass%.

The isocyanate-terminated urethane prepolymer is added and mixed in the aqueous solution cooled at room temperature. An addition mass of the isocyanate-terminated urethane prepolymer is conventionally about 3-20 parts by mass to 100 parts by mass of the aqueous solution. The machine and degree for mixing are decided so as to emulsify the isocyanate-terminated urethane prepolymer in the water. It is used a homomixer, a homogenizer, a high-pressure homogenizer or an ultrasonic homogenizer as the machine for mixing. Using the homomixer, the degree for mixing is about 5 minutes at about 8000 rpm. Adding and mixing are conventionally executed at room temperature, but may be executed at a little heating temperature so as to mix easily.

A trifunctional or more functional amine is added in the oil-in-water emulsion. The trifunctional or more functional amine is used to obtain polyurethane having network structure by three-dimensionally polymerizing the isocyanate-terminated urethane prepolymer as described in paragraph 0011. It is used various poly-functional amine as the trifunctional or more functional amine. The poly-functional amine is added as it is or as solution dissolving it in a solvent such as water.

An addition mass of the poly-functional amine is preferably chemical equivalent to the isocyanate group of the isocyanate-terminated urethane prepolymer in the oil-in-water emulsion. That is, the poly-functional amine is preferably added such as an amino group of the poly-functional amine is chemical equivalent to the isocyanate group of the isocyanate-terminated urethane prepolymer. Thereby, the isocyanate group reacts with the amino group to three-dimensionally polymerize. The addition mass of the poly-functional amine may be from 0.8 to less than the chemical equivalent to restraint three-dimensional polymerization. Furthermore, the addition mass of the poly-functional amine may be to 1.2 from more than the chemical equivalent to be densely three-dimensional polymerization.

Not only the poly-functional amine such as the trifunctional or more functional amine but also a difunctional amine or polyalcohol may be added with the poly-functional amine to react with isocyanate group.

Using the difunctional amine or polyalcohol, it may be controlled to three-dimensionally polymerize the isocyanate-terminated urethane prepolymer.

Three-dimensionally polymerizing the isocyanate-terminated urethane prepolymer in oil droplets with the addition of the poly-functional amine, the oil-in-water emulsion is conventionally heated to quicken the reaction of the polymerization. The temperature of the oil-in-water emulsion is decided on the reactivity of the poly-functional amine. For example, using the 3,3'-diaminodipropylamine as the poly-functional amine, the oil-in-water emulsion is heated at about 80 °C, then the three-dimensional polymerization has finished for about 20 hours.

After the three-dimensional polymerization is finished, the oil droplets in the oil-in-water emulsion are converted to the polyurethane spheres. That is, the polyurethane spheres are dispersed to be in a dispersion. The dispersant in the dispersion is removed. The method of removing the dispersant is the following. The polyurethane spheres are collected with filtering or centrifugal separating the dispersion. The collected polyurethane spheres are dispersed in a water again. The polyurethane spheres are again collected with filtering or centrifugal separating the second dispersion. By repeating the above method, it is obtained an aqueous polyurethane dispersion in which the polyurethane spheres disperse as dispersion medium of water.

It is prepared the aqueous silica dispersion in which the hydrophilic fine silica powders disperse into a water. It is easily prepared to add and mix the hydrophilic fine silica powders in the water. It is decided the condition of adding and mixing or the content of the powders so as to disperse the powders informally in the water, not cohering. For example, using the trade mark ┌Aerosil ^{®} 200┘ which are the hydrophilic fine silica powders and dispersing them in a content of 5 mass%, the aqueous silica dispersion is easily obtained by mixing with a water for 10 minutes at 10000 rpm by a homomixer.

Mixing the above aqueous silica dispersion with the above aqueous polyurethane dispersion, the mixed aqueous dispersion is obtained. The mixing ratio of the both is decided on the ratio of the solid content of the both. Mixing the both, it is preferable to add a water so as to prevent that the hydrophilic fine silica powders and polyurethane spheres cohere. The addition mass of the water is optionally decided, conventionally being about equal to the mass of the aqueous polyurethane dispersion. Adding the water, it is preferable to mix so as to prevent coherence.

The mixed aqueous dispersion is dried with the spray-dryer having nozzles or disks. Using the spray-dryer having nozzles, the mixed aqueous dispersion is spray-dried through each nozzle in the high temperature atmosphere to dry. Using the spray-dryer having disks, the mixed aqueous dispersion is dropped on each rotating disk and spray-dried on the centrifugal force of each disk in the high temperature atmosphere to dry. The spray-drying in the invention is characterized by spraying in remaining the form of each polyurethane sphere in the mixed aqueous dispersion, that is, not atomizing the polyurethane sphere. Because the polyurethane sphere is three-dimensionally polymerized in the mixed aqueous dispersion. Spraying the mixed aqueous dispersion, it is sprayed the polyurethane spheres and the hydrophilic fine silica powders with the water. Drying the water, the hydrophilic fine silica powders adhere onto each surface of the polyurethane spheres. The polyurethane spheres being softer, the hydrophilic fine silica powders adhere on the surface in implanting.

The condition of spraying is not strict because the polyurethane spheres are not atomized at spraying. It is not strict the condition of the pressure, centrifugal force and aperture of the nozzle for atomization. Therefore, the spray-dryer having nozzles or disks may be used. The condition of the high temperature atmosphere to dry after spraying is decided so as to vapor the spraying water. The condition is conventional, for example, the inlet of a hot air may be the temperature of about 140°C and the temperature of about 70°C into the dryer. Spraying and drying the polyurethane spheres, each body of the polyurethane particles is formed with one of them or joining two or more. The polyurethane sphere being softer or more stick surface, the body of the polyurethane particles tends to be formed with two or more of the polyurethane spheres. The polyurethane spheres being harder, the body of the polyurethane particles tends to be formed with one of the polyurethane spheres.

Spray-drying the mixed aqueous dispersion, the polyurethane sphere is converted to the body of the polyurethane particle, covering the surface of the body with the hydrophilic fine silica powders. The hydrophilic fine silica powders adhere on the body. Being softer the polyurethane sphere or the body of the polyurethane particle, the hydrophilic fine silica powders adhere on the body in implanting. An average particle size of the polyurethane particles is decided on an average particle size of the polyurethane sphere in the mixed aqueous dispersion, a softness or stickiness of the body or the condition of spray-drying. The average particle size of the polyurethane particles is conventionally about 1-500µ m. The average particle size of the polyurethane particles means to be median size on volume basis. The average particle size is measured with the laser scattering type particle size distribution analyzer which is sold as trade name ┌LA-920 ┘ from HORIBA, Ltd in Japan, after dispersing the polyurethane particles in a deionized water.

The polyurethane particle is used as an oil absorbent particle, and the polyurethane particles are as an oil absorbent agent. The oil absorbent agent is used as a component for cosmetics to make-up, care skin, skin care cosmetics, deodorize, protect ultraviolet and treat hair because the oil absorbent agent superiorly absorbs sebum. Furthermore, the oil absorbent agent is used as a component for waste oil disposal because the oil absorbent agent superiorly absorbs oils such as oleic acid. The polyurethane particles superiorly absorb a perfumed oil. Therefore, the polyurethane particles absorbed the perfumed oil may be used as a perfume which is able to use for a long time, gradually releasing the perfumed oil therein. Furthermore, absorbing an oil-based chemical component such as oil-based agricultural chemical component, oil-based attractant component or oil-based repellent component, and scattering in an environment, chemicals comprising the absorbed polyurethane particles may be used for a long time, gradually releasing the oil-based chemical component.

### Advantageous Effects of invention

The polyurethane particles are smooth because of covering on the body of the polyurethane particle with the hydrophilic fine silica powders. Therefore, the polyurethane particles are easily flowed for a long time because they are not cohered at keeping or transporting. The body of the polyurethane particle is formed by three-dimensionally polymerizing the isocyanate-terminated urethane prepolymer with the trifunctional or more functional amines. Further, the isocyanate-terminated urethane prepolymer is obtained by reacting the polyisocyanates and the polyols including the poly(tetramethylene ether)glycol. Therefore, the polyurethane particle superiorly absorbs the sebums and oils such as oleic acid because the body of the polyurethane particle comprises the poly(tetramethylene ether)glycol as an element of polymerization. Furthermore, absorbing the oils, the polyurethane particle is not solved but only swelled because the isocyanate-terminated urethane prepolymer comprising the element of the poly(tetramethylene ether)glycol is three-dimensionally polymerized with the trifunctional or more functional amines. Therefore, the body of the polyurethane particle remains to be covered with the hydrophilic fine silica powders, and the polyurethane particles are smooth, not sticky or liquidizing.

The method of producing polyurethane particles comprises of spraying the mixed aqueous dispersion in the high temperature atmosphere. Further, the mixed aqueous dispersion is obtained by dispersing the hydrophilic fine silica powders and the three-dimensionally polymerized polyurethane spheres in the water. Therefore, spraying the mixed aqueous dispersion, the polyurethane spheres are not atomizes because of the three-dimensionally polymerization. Adjusting the size of the polyurethane sphere which is then formed, it is obtained the polyurethane particle having the desired size after spraying. Furthermore, spraying the mixed aqueous dispersion, the hydrophilic fine silica powders are sprayed with the polyurethane spheres. Then, the hydrophilic fine silica powders and the water exist around the polyurethane sphere. Vaporizing the water by drying, the hydrophilic fine silica powders collide on the surface of the polyurethane sphere. Therefore, the polyurethane sphere which is the body of the polyurethane particle is covered with the hydrophilic fine silica powders. By the method, it is obtained the smooth polyurethane particles which are difficult to cohere.

### Examples

### Example 1

### [Preparing an isocyanate-terminated urethane prepolymer]

20 parts by mass of ethyl acetate and 300 parts by mass of poly(tetramethylene ether)glycol having a number average molecular weight of 1000 were charged in a four-necked flask fitted with a stirrer, nitrogen inlet, thermometer and condenser. 104.6 parts by mass of isophorone diisocyanate and 0.04 parts by mass of dibutyl tin dilaurate were added and reacted under a nitrogen stream at 75-80 °C for 5 hours to obtain the isocyanate-terminated urethane prepolymer containig 3.6% of isocyanate group.

### [Preparing an oil-in-water emulsion]

900 parts by mass of deionized water and 100 parts by mass of polyvinyl alcohol having a saponification degree of 86.5-89.0 mol% as a dispersant which was sold as a trade name of PVA-205 by Kuraray Co., Ltd. were charged and heated at 90 °C to obtain an aqueous solution of polyvinyl alcohol. 100 parts by mass of the above prepared isocyanate-terminated urethane prepolymer were added in the aqueous solution of polyvinyl alcohol, and they were mixed at 8000 rpm for 5 minutes with a homomixer to obtain the oil-in-water emulsion in which oils are consisted of the isocyanate-terminated urethane prepolymer.

### [Preparing an aqueous polyurethane dispersion]

35.7 parts by mass of 10% aqueous solution of 3,3'-diaminodipropylamine were added in the above oil-in-water emulsion. They were heated at 80 °C with stirring, remained at the temperature and reacted for 20 hours. The isocyanate-terminated urethane prepolymer reacted with the 3,3'-diaminodipropylamine to be three-dimensionally polymerized, and polyurethane spheres were formed. The polyurethane spheres were sunk and collected with a centrifugation. The polyurethane spheres were again dispersed in a water and were sunk and collected with a centrifugation. Sixth dispersing and collecting were carried out to remove the polyvinyl alcohol as the dispersant from the water. It is obtained the aqueous polyurethane dispersion comprising the polyurethane spheres of 40% by mass.

### [Preparing an aqueous silica dispersion]

95 parts by mass of deionized water were charged and stirred at 10000 rpm in a homomixer. 5 parts by mass of hydrophilic fine silica powders having an average particle size of 20 nm which were sold as a trade name of AEROSIL 200 by Nippon Aerosil Co., Ltd. were added in the deionized water and mixed for 10 minutes to obtain the aqueous silica dispersion.

### [Preparing a mixed aqueous dispersion]

100 parts by mass of the above aqueous polyurethane dispersion, 80 parts by mass of the above aqueous silica dispersion and 110 parts by mass of deionized water were mixed to obtain the mixed aqueous dispersion.

### [Producing polyurethane particles]

Spray-drying the above mixed aqueous dispersion with a spray-dryer which was sold as a trade name of L-8i by Ohkawara Kakohki Co., Ltd., the polyurethane particles were obtained. Each condition of the spray-drying was as the following. A pressure of spraying was 0.3MPa, a temperature of an inlet of a hot air was 140 °C and a temperature into a dryer was 70°C. Vaporizing the water in the mixed aqueous dispersion by the spray-drying, the polyurethane spheres were converted to bodies of the polyurethane particles and the hydrophilic fine silica powders were adhered to each surface of the bodies of the polyurethane particles in implanted. Therefore, each body of the polyurethane particles was covered with the hydrophilic fine silica powders to be the polyurethane particle. The polyurethane particles were not cohered and smooth because of separating from each one. An average particle size of the polyurethane particles is about 12 µm.

### Example 2

### [Preparing an isocyanate-terminated urethane prepolymer]

The isocyanate-terminated urethane prepolymer containing 2.5% of isocyanate group was obtained with the same method of the Example 1 except that a poly(tetramethylene ether)glycol having a number average molecular weight of 3000 was used in place of the number average molecular weight of 1000 and 45.0 parts by mass of isophorone diisocyanate were used in place of the 104.6 parts by mass.

### [Preparing an oil-in-water emulsion]

The oil-in-water emulsion was obtained with the same method of the Example 1 except that the above isocyanate-terminated urethane prepolymer was used.

### [Preparing an aqueous polyurethane dispersion]

The aqueous polyurethane dispersion was obtained with the same method of the Example 1 except that the above oil-in-water emulsion was used and 24.5 parts by mass of 10% aqueous solution of 3,3'-diaminodipropylamine were used in place of the 35.7 parts by mass.

### [Preparing an aqueous silica dispersion]

The aqueous silica dispersion was obtained with the same method of the Example 1.

### [Preparing a mixed aqueous dispersion]

The mixed aqueous dispersion was obtained with the same method of the Example 1 except that the above aqueous polyurethane dispersion was used.

### [Producing polyurethane particles]

The polyurethane particles were obtained with the same method of the Example 1 except that the above mixed aqueous dispersion was used.

The properties of the polyurethane particles were the same as them of the Example 1. An average particle size of the polyurethane particles is about 15 µm.

### Example 3

### [Preparing an isocyanate-terminated urethane prepolymer]

The isocyanate-terminated urethane prepolymer was obtained with the same method of the Example 1.

### [Preparing another isocyanate-terminated urethane prepolymer]

20 parts by mass of ethyl acetate and 300 parts by mass of polycarbonatediol having a number average molecular weight of 2000 which was sold as a trade name PCDL T-6002 by Asahi Kasei Chemicals Co. were charged in a four-necked flask fitted with a stirrer, nitrogen inlet, thermometer and condenser. 67.4 parts by mass of isophorone diisocyanate and 0.04 parts by mass of dibutyl tin dilaurate were added and reacted under a nitrogen stream at 75-80 °C for 5 hours to obtain the another isocyanate-terminated urethane prepolymer containing 3.5% of isocyanate group.

### [Preparing an oil-in-water emulsion]

The oil-in-water emulsion was obtained with the same method of the Example 1 except that 100 parts by mass of a mixture which consisted of 52 parts by mass of the isocyanate-terminated urethane prepolymer obtained in the Example 1 and 48 parts by mass of the above another isocyanate-terminated urethane prepolymer were used.

### [Preparing an aqueous polyurethane dispersion]

The aqueous polyurethane dispersion was obtained with the same method of the Example 1 except that the above oil-in-water emulsion was used and 34.6 parts by mass of 10% aqueous solution of 3,3'-diaminodipropylamine were used in place of the 35.7 parts by mass.

### [Preparing an aqueous silica dispersion]

The aqueous silica dispersion was obtained with the same method of the Example 1.

### [Preparing a mixed aqueous dispersion]

The mixed aqueous dispersion was obtained with the same method of the Example 1 except that the above aqueous polyurethane dispersion was used.

### [Producing polyurethane particles]

The polyurethane particles were obtained with the same method of the Example 1 except that the above mixed aqueous dispersion was used.

The properties of the polyurethane particles were the same as them of the Example 1. An average particle size of the polyurethane particles is about 14 µm.

### Example 4

### [Preparing an isocyanate-terminated urethane prepolymer]

20 parts by mass of ethyl acetate, 150 parts by mass of poly(tetramethylene ether)glycol having a number average molecular weight of 1000 and 150 parts by mass of polycarbonatediol having a number average molecular weight of 2000 which was sold as a trade name PCDL T-6002 by Asahi Kasei Chemicals Co. were charged in a four-necked flask fitted with a stirrer, nitrogen inlet, thermometer and condenser.

85.4 parts by mass of isophorone diisocyanate and 0.1 parts by mass of 1,8-diazabicyclo[5.4.0]undec-7-ene were added and reacted under a nitrogen stream at 75-80 °C for 5 hours to obtain the isocyanate-terminated urethane prepolymer containing 3.5% of isocyanate group.

### [Preparing an oil-in-water emulsion]

The oil-in-water emulsion was obtained with the same method of the Example 1 except that the above isocyanate-terminated urethane prepolymer was used.

### [Preparing an aqueous polyurethane dispersion]

The aqueous polyurethane dispersion was obtained with the same method of the Example 1 except that the above oil-in-water emulsion was used and 34.7 parts by mass of 10% aqueous solution of 3,3'-diaminodipropylamine were used in place of the 35.7 parts by mass.

### [Preparing an aqueous silica dispersion]

The aqueous silica dispersion was obtained with the same method of the Example 1.

### [Preparing a mixed aqueous dispersion]

The mixed aqueous dispersion was obtained with the same method of the Example 1 except that the above aqueous polyurethane dispersion was used.

### [Producing polyurethane particles]

The polyurethane particles were obtained with the same method of the Example 1 except that the above mixed aqueous dispersion was used.

The properties of the polyurethane particles were the same as them of the Example 1. An average particle size of the polyurethane particles is about 13 µm.

### Comparative Example 1

### [Preparing another isocyanate-terminated urethane prepolymer]

Another isocyanate-terminated urethane prepolymer was obtained with the same method of the Example 3.

### [Preparing an oil-in-water emulsion]

The oil-in-water emulsion was obtained with the same method of the Example 1 except that the above another isocyanate-terminated urethane prepolymer was used.

### [Preparing an aqueous polyurethane dispersion]

The aqueous polyurethane dispersion was obtained with the same method of the Example 1 except that the above oil-in-water emulsion was used and 34.6 parts by mass of 10% aqueous solution of 3,3'-diaminodipropylamine were used in place of the 35.7 parts by mass.

### [Preparing an aqueous silica dispersion]

The aqueous silica dispersion was obtained with the same method of the Example 1.

### [Preparing a mixed aqueous dispersion]

The mixed aqueous dispersion was obtained with the same method of the Example 1 except that the above aqueous polyurethane dispersion was used.

### [Producing polyurethane particles]

The polyurethane particles were obtained with the same method of the Example 1 except that the above mixed aqueous dispersion was used.

The properties of the polyurethane particles were the same as them of the Example 1. An average particle size of the polyurethane particles is about 16 µm.

Oil adsorption capacity was measured with an artificial sebum, oleic acid and squalane concerning the polyurethane particles obtained by the methods of Example 1-4 and Comparative Example 1. The oil adsorption capacity was measured by the following method. 0.2 grams of the polyurethane particles were dipped in 10 ml of the artificial sebum and left for 3 days under a room temperature. After the polyurethane particles sufficiently absorbed the artificial sebum, an excess quantity of the artificial sebum was removed with a vacuum filtration method for 10 minutes by using a membrane filter. After removing, the weight of the polyurethane particles absorbing the artificial sebum was measured. The increase of the polyurethane particles was the oil adsorption capacity. The oil adsorption capacity was calculated with a unit of g/100g. Furthermore, the artificial sebum consisted of 29 parts by mass of trioleic acid, 28.5 parts by mass of oleic acid, 18.5 parts by mass of oleyl oleate, 14 parts by mass of squalane, 7 parts by mass of cholesterol and 3 parts by mass of myristyl myristate. Each adsorption capacity of the oleic acid and squalane was measured with the above method. The results were showed in the table 1.

**[Table 1]**

| | Example | | | | Compar. Example |
|---|---|---|---|---|---|
| | 1 | 2 | 3 | 4 | 1 |
| artificial sebum | 218 | 293 | 119 | 129 | 25 |
| oleic acid | 342 | 439 | 202 | 224 | 54 |
| squalane | 20 | 11 | 22 | 21 | 24 |

The polyurethane particles obtained by the methods of Example 1-4 selectively absorbed the artificial sebum and oleic acid more than the polyurethane particles obtained by the method of the Comparative Example 1 as shown in the table 1. This reason may be the followings.

The polyurethane particles were produced by using the isocyanate-terminated urethane prepolymers which were obtained by reacting the polyisocyanates and the polyols including the poly(tetramethylene ether)glycol in the Example 1-4. The polyurethane particles were produced by using the isocyanate-terminated urethane prepolymer which was obtained by reacting the polyisocyanate and the polyols except a poly(tetramethylene ether) glycol in the Comparative Example 1.

## Claims

1. A polyurethane particle the surface of the body of which is covered with hydrophilic fine silica powders, wherein;
the body of the polyurethane particle is obtained by three-dimensionally polymerizing an isocyanate-terminated urethane prepolymer with trifunctional or more functional amines, and the isocyanate-terminated urethane prepolymer is obtained by reacting polyisocyanates and polyols including a poly(tetramethylene ether)glycol.

2. The polyurethane particle according to the claim 1, wherein;
the surface of the body of the polyurethane particle is covered with hydrophilic fine silica powders which are implanted on the surface of the body.

3. The polyurethane particle according to the claim 1, wherein; a number average molecular weight of poly(tetramethylene ether) glycol is 650-3000.

4. The polyurethane particle according to the claim 1, wherein;
an isophorone diisocyanate is used as the polyisocyanates and the poly(tetramethylene ether)glycol is used as the polyols.

5. The polyurethane particle according to the claim 1, wherein;
a 3,3'-diaminodipropylamine is used as trifunctional amines.

6. The polyurethane particle according to the claim 1, wherein;
a particle size of the polyurethane particle is 30 or more times larger than a particle size of the hydrophilic fine silica powder.

7. An oil absorbent agent comprising a large number of the polyurethane particles defined in the claim 1.

8. A perfume comprising a large number of the polyurethane particles defined in the claim 1 which hold a perfumed oil therein.

9. Chemicals comprising a large number of the polyurethane particles defined in the claim 1 which hold therein an oil-based chemical component selected from the group consisting of an oil-based agricultural chemical component, an oil-based attractant component and an oil-based repellent component.

10. A method for producing polyurethane particles comprising;
preparing hydrophilic fine silica powders,
preparing an isocyanate-terminated urethane prepolymer obtained by reacting polyisocyanates and polyols including a poly(tetramethylene ether)glycol,
preparing polyurethane spheres obtained by three-dimensionally polymerizing the isocyanate-terminated urethane prepolymer with trifunctional or more functional amines,
preparing a mixed aqueous dispersion by dispersing the hydrophilic fine silica powders and the polyurethane spheres in a water, and
spraying the mixed aqueous dispersion in a high temperature atmosphere, thereby
vaporizing the water in the mixed aqueous dispersion, converting the polyurethane spheres to bodies of the polyurethane particles, and covering each surface of the bodies of the polyurethane particles with the hydrophilic fine silica powders.

11. A method for producing polyurethane particles comprising the following processes;
a process to obtain an isocyanate-terminated urethane prepolymer by reacting polyisocyanates and polyols including a poly(tetramethylene ether)glycol,
a process to obtain an oil-in-water emulsion by adding and mixing the isocyanate-terminated urethane prepolymer to an aqueous solution in which a dispersant dissolves,
a process to obtain polyurethane spheres in the oil-in-water emulsion by that the isocyanate-terminated urethane prepolymer in oil droplets is three-dimensionally polymerized by adding the trifunctional or more functional amines in the oil-in-water emulsion,
a process to obtain an aqueous polyurethane dispersion in which the polyurethane spheres disperse by removing the dispersant from the oil-in-water emulsion.
a process to obtain a mixed aqueous dispersion by mixing the aqueous polyurethane dispersion with an aqueous silica dispersion in which hydrophilic fine silica powders disperse into a water, and
a process to spray-dry the mixed aqueous dispersion in a high temperature atmosphere with a spray-dryer, thereby
vaporizing the water in the mixed aqueous dispersion, converting the polyurethane spheres to bodies of the polyurethane particles, and covering each surface of the bodies of the polyurethane particles with the hydrophilic fine silica powders.

12. The method for producing polyurethane particles according to the claim 10 or 11, wherein;
each surface of the bodies of the polyurethane particles is covered with the hydrophilic fine silica powders which are implanted on each surface of the bodies.

13. The method for producing polyurethane particles according to the claim 11, wherein;
a polyvinyl alcohol is used as the dispersant.

14. The method for producing polyurethane particles according to the claim 11, wherein;
an average particle size of the hydrophilic fine silica powders is 5-40 nm, and an average particle size of the polyurethane particles is 1-500 µm.

15. An oil absorbent agent comprising the polyurethane particles obtained by the method defined in the claim 10 or 11.

16. Chemicals comprising the polyurethane particles obtained by the method defined in the claim 10 or 11, which hold therein an oil-based chemical component selected from the group consisting of an oil-based agricultural chemical component, an oil-based attractant component and an oil-based repellent component.

## Patentansprüche

1. Polyurethanteilchen, dessen Körperoberfläche mit hydrophilen feinen Silicapulvern bedeckt ist, wobei:
der Körper des Polyurethanteilchens durch dreidimensionales Polymerisieren eines Isocyanat-terminierten Urethan-Präpolymers mit trifunktionellen oder mehr-funktionellen Aminen erhalten wird, und das Isocyanat-terminierte Urethan-Präpolymer durch Umsetzen von Polyisocyanaten und Polyolen, einschliesslich Poly(tetramethylenether)glykol, erhalten wird.

2. Polyurethanteilchen gemäss Anspruch 1, wobei:
die Körperoberfläche des Polyurethanteilchens mit hydrophilen feinen Silicapulvern bedeckt ist, die auf der Körperoberfläche implantiert sind.

3. Polyurethanteilchen gemäss Anspruch 1, wobei:
das zahlengemittelte Molekulargewicht von Poly(tetramethylenether)glykol 650 bis 3.000 beträgt.

4. Polyurethanteilchen gemäss Anspruch 1, wobei:
ein Isophorondiisocyanat als Polyisocyanate und das Poly(tetramethylenether)glykol als Polyole verwendet werden.

5. Polyurethanteilchen gemäss Anspruch 1, wobei:
ein 3,3'-Diaminodipropylamin als trifunktionelle Amine verwendet wird.

6. Polyurethanteilchen gemäss Anspruch 1, wobei:
die Teilchengrösse der Polyurethanteilchen 30-fach oder mehr grösser als die Teilchengrösse des hydrophilen feinen Silicapulvers ist.

7. Ölabsorptionsmittel, umfassend eine grosse Anzahl der in Anspruch 1 definierten Polyurethanteilchen.

8. Parfüm, umfassend eine grosse Anzahl der in Anspruch 1 definierten Polyurethanteilchen, die darin ein Parfümöl enthalten.

9. Chemikalie, umfassend eine grosse Anzahl der in Anspruch 1 definierten Polyurethanteilchen, die darin eine Öl-basierte chemische Komponente, ausgewählt aus der Gruppe bestehend aus einer Öl-basierten agrar-chemischen Komponente, einer Öl-basierten Lockmittelkomponente und einer Öl-basierten Repellens- Komponente, enthalten.

10. Verfahren zur Herstellung von Polyurethanteilchen, umfassend:
Herstellung von hydrophilen feinen Silicapulvern,
Herstellung eines Isocyanat-terminierten Urethan-Präpolymers, erhalten durch Umsetzen von Polyisocyanaten und Polyolen, einschliesslich Poly(tetramethylenether)glykol,
Herstellung von Polyurethankugeln, erhalten durch dreidimensionales Polymerisieren des Isocyanat-terminierten Urethan-Präpolymers mit trifunktionellen oder mehr-funktionellen Aminen,
Herstellung einer gemischten wässrigen Dispersion durch Dispergieren der hydrophilen feinen SilicaPulver und der Polyurethankugeln in Wasser, und
Sprühen der gemischten wässrigen Dispersion in einer Hochtemperaturatmosphäre, wodurch
das Wasser in der gemischten wässrigen Dispersion verdampft wird, Umwandeln der Polyurethankugeln zu Körpern der Polyurethanteilchen und Bedecken jeder Oberfläche der Körper der Polyurethanteilchen mit den hydrophilen feinen Silicapulvern.

11. Verfahren zur Herstellung von Polyurethanteilchen, umfassend die nachstehenden Verfahren:
ein Verfahren zum Erhalt eines Isocyanat-terminierten Urethan-Präpolymers durch Umsetzen von Polyisocyanaten und Polyolen, einschliesslich einem Poly(tetramethylenether)glykol,
ein Verfahren zum Erhalt einer Öl-in-Wasser-Emulsion durch Zugeben und Mischen des Isocyanat-terminierten Urethan-Präpolymers zu einer wässrigen Lösung, in der sich ein Dispersionsmittel auflöst,
ein Verfahren zum Erhalt von Polyurethankugeln in der Öl-in-Wasser-Emulsion, bei dem das Isocyanat-terminierte Urethan-Präpolymer in Öltröpfchen durch Zugabe der trifunktionellen oder mehr-funktionellen Amine zu der Öl-in-Wasser-Emulsion dreidimensional polymerisiert wird,
ein Verfahren zum Erhalt einer wässrigen Polyurethandispersion, in der die Polyurethankugeln durch Entfernen des Dispersionsmittels aus der Öl-in-Wasser-Emulsion dispergieren,
ein Verfahren zum Erhalt einer gemischten wässrigen Dispersion durch Mischen der wässrigen Polyurethandispersion mit einer wässrigen Silicadispersion, in der die hydrophilen feinen Silicapulver in Wasser dispergieren, und
ein Verfahren zum Sprühtrocknen der gemischten wässrigen Dispersion in einer Hochtemperaturatmosphäre mit einem Sprühtrockner, wodurch
das Wasser in der gemischten wässrigen Dispersion verdampft, Umwandeln der Polyurethanteilchen zu Körpern der Polyurethanteilchen und Bedecken jeder Körperoberfläche der Polyurethanteilchen mit den hydrophilen feinen Silicapulvern.

12. Verfahren zur Herstellung von Polyurethanteilchen gemäss Anspruch 10 oder 11, wobei:
jede Körperoberfläche der Polyurethanteilchen mit den hydrophilen feinen Silicapulvern, die auf der Oberfläche der Körper implantiert sind, bedeckt ist.

13. Verfahren zur Herstellung von Polyurethanteilchen gemäss Anspruch 11, wobei:
ein Polyvinylalkohol als Dispersionsmittel verwendet wird.

14. Verfahren zur Herstellung von Polyurethanteilchen gemäss Anspruch 11, wobei:
die durchschnittliche Teilchengrösse der hydrophilen feinen Silicapulver 5 bis 40 nm beträgt und die durchschnittliche Teilchengrösse der Polyurethanteilchen 1 bis 500 µm beträgt.

15. Ölabsorptionsmittel, umfassend die Polyurethanteilchen, die nach dem in Anspruch 10 oder 11 definierten Verfahren erhalten wurden.

16. Chemikalie, umfassend die Polyurethanteilchen, die nach dem in Anspruch 10 oder 11 definierten Verfahren erhalten wurden, die darin eine Öl-basierte Komponente, ausgewählt aus der Gruppe bestehend aus einer Öl-basierten agrar-chemischen Komponente, einer Öl-basierten Lockmittelkomponente und einer Ölbasierten Repellens-Komponente, enthalten.

## Revendications

1. Particule de polyuréthane dont la surface du corps est recouverte de fines poudres de silice hydrophile, dans laquelle :
le corps de la particule de polyuréthane est obtenu par polymérisation tridimensionnelle d'un prépolymère d'uréthane à terminaison isocyanate avec des amines trifonctionnelles ou ayant plus de fonctions et le prépolymère d'uréthane à terminaison isocyanate est obtenu en faisant réagir des polyisocyanates et des polyols comprenant un poly(éther de tétraméthylène)glycol.

2. Particule de polyuréthane selon la revendication 1, dans laquelle :
la surface du corps de la particule de polyuréthane est couverte de fines poudres de silice hydrophile qui sont implantées sur la surface du corps.

3. Particule de polyuréthane selon la revendication 1, dans laquelle :
un poids moléculaire moyen en nombre du poly(éther de tétraméthylène)glycol est de 650 à 3000.

4. Particule de polyuréthane selon la revendication 1, dans laquelle :
un diisocyanate d'isophorone est utilisé comme polyisocyanates et le poly(éther de tétraméthylène)glycol est utilisé comme polyols.

5. Particule de polyuréthane selon la revendication 1, dans laquelle :
une 3,3'-diaminodipropylamine est utilisée comme amines trifonctionnelles.

6. Particule de polyuréthane selon la revendication 1, dans laquelle :
une taille de particule de la particule de polyuréthane est 30 fois plus grande, ou plus, qu'une taille de particule de la fine poudre de silice hydrophile.

7. Agent absorbant l'huile, comprenant un grand nombre de particules de polyuréthane définies dans la revendication 1.

8. Parfum comprenant un grand nombre de particules de polyuréthane définies dans la revendication 1 qui retiennent dans celles-ci une huile parfumée.

9. Produits chimiques comprenant un grand nombre de particules de polyuréthane définies dans la revendication 1 qui retiennent dans celles-ci un composant chimique à base d'huile choisi dans le groupe constitué d'un composant chimique agricole à base d'huile, un composant attractif à base d'huile et un composant répulsif à base d'huile.

10. Procédé de production de particules de polyuréthane comprenant :
la préparation de fines poudres de silice hydrophile,
la préparation d'un prépolymère d'uréthane à terminaison isocyanate obtenu en faisant réagir des polyisocyanates et des polyols comprenant un poly(éther de tétraméthylène)glycol,
la préparation de sphères de polyuréthane obtenues par polymérisation tridimensionnelle du prépolymère d'uréthane à terminaison isocyanate avec des amines trifonctionnelles ou ayant plus de fonctions,
la préparation d'une dispersion aqueuse mixte par dispersion des fines poudres de silice hydrophile et des sphères de polyuréthane dans l'eau, et
la pulvérisation de la dispersion aqueuse mixte dans une atmosphère à température élevée et, ainsi,
la vaporisation de l'eau dans la dispersion aqueuse mixte, la conversion des sphères de polyuréthane en corps de particules de polyuréthane, et le recouvrement de chaque surface des corps de particules de polyuréthane avec les fines poudres de silice hydrophile.

11. Procédé de production de particules de polyuréthane comprenant les procédés suivants :
un procédé d'obtention d'un prépolymère d'uréthane à terminaison isocyanate en faisant réagir des polyisocyanates et des polyols comprenant un poly(éther de tétraméthylène)glycol,
un procédé d'obtention d'une émulsion huile dans l'eau en ajoutant et en mélangeant le prépolymère d'uréthane à terminaison isocyanate dans une solution aqueuse dans laquelle un dispersant se dissout,
un procédé d'obtention de sphères de polyuréthane dans l'émulsion huile dans eau par lequel le prépolymère d'uréthane à terminaison isocyanate dans des gouttelettes d'huile est polymérisé de façon tridimensionnelle en ajoutant les amines trifonctionnelles ou ayant plus de fonctions dans l'émulsion huile dans eau,
un procédé d'obtention d'une dispersion de polyuréthane aqueuse dans laquelle les sphères de polyuréthane se dispersent en éliminant le dispersant de l'émulsion huile dans eau,
un procédé d'obtention d'une dispersion aqueuse mixte en mélangeant la dispersion de polyuréthane aqueuse avec une dispersion de silice aqueuse dans laquelle les fines poudres de silice hydrophiles se dispersent dans l'eau, et
un procédé de séchage par pulvérisation de la dispersion aqueuse mixte dans une atmosphère à haute température avec un séchoir par pulvérisation et, ainsi,
la vaporisation de l'eau dans la dispersion aqueuse mixte, la conversion des sphères de polyuréthane en corps des particules de polyuréthane et le recouvrement de chaque surface des corps des particules de polyuréthane avec les fines poudres de silice hydrophile.

12. Procédé de production de particules de polyuréthane selon la revendication 10 ou 11, dans lequel :
chaque surface des corps des particules de polyuréthane est recouverte des fines poudres de silice hydrophile qui sont implantées sur chaque surface des corps.

13. Procédé de production des particules de polyuréthane selon la revendication 11, dans lequel :
un alcool de polyvinyle est utilisé comme dispersant.

14. Procédé de production de particules de polyuréthane selon la revendication 11, dans lequel :
une taille moyenne de particules des fines poudres de silice hydrophile est de 5 à 40 nm et une taille moyenne de particules des particules de polyuréthane est de 1 à 500 µm.

15. Agent absorbant l'huile comprenant les particules de polyuréthane obtenues par le procédé défini dans la revendication 10 ou 11.

16. Produits chimiques comprenant les particules de polyuréthane obtenues par le procédé défini dans la revendication 10 ou 11, qui retiennent dans celles-ci un composant chimique à base d'huile choisi dans le groupe constitué d'un composant chimique agricole à base d'huile, un composant attractif à base d'huile et un composant répulsif à base d'huile.
